(19) Europäisches Patentamt
European Patent Office
Office européen des brevets

(11) **EP 4 640 144 A1**

(12) **EUROPEAN PATENT APPLICATION**

(43) Date of publication:
**29.10.2025 Bulletin 2025/44**

(21) Application number: **24171963.2**

(22) Date of filing: **23.04.2024**

(51) International Patent Classification (IPC):
**A61B 5/021** (2006.01)   **A61B 5/00** (2006.01)

(52) Cooperative Patent Classification (CPC):
**A61B 5/021; A61B 5/02108; A61B 5/7235;
A61B 5/7253; A61B 5/7278**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Koninklijke Philips N.V.
5656 AG Eindhoven (NL)**

(72) Inventors:
• **MUEHLSTEFF, Jens
5656 AG Eindhoven (NL)**
• **WIJSHOFF, Ralph Wilhelm Christianus Gemma
Rosa
5656 AG Eindhoven (NL)**
• **LAU, Kevin Daniel Seng Hung
5656 AG Eindhoven (NL)**

(74) Representative: **Philips Intellectual Property &
Standards
High Tech Campus 52
5656 AG Eindhoven (NL)**

(54) **AORTIC PULSE WAVEFORM ESTIMATION**

(57)    A method for inferring a central (e.g. aortic) blood pulse waveform from a peripheral pulse waveform. The method comprises configuring a transformation operation from the peripheral to central waveforms based on one or more waveform features of the measured peripheral pulse waveform.

FIG. 3

**Description**

FIELD OF THE INVENTION

The present invention relates to the field of non-invasive hemodynamic monitoring.

BACKGROUND OF THE INVENTION

For inference of hemodynamic parameters such as Stroke Volume (SV) or Cardiac Output (CO), one set of methods involves estimating a Blood Pressure (BP) pulse waveform at the location of the ascending aorta. From this pulse waveform, hemodynamic parameters can be inferred.

However, BP waveforms are typically measured peripherally, i.e. via a measurement at a peripheral vascular location. This can be performed invasively (with an invasive arterial line at the radial artery), or non-invasively, for example by using a vascular clamp applied to the finger, or by using a non-invasive blood pressure (NIBP) measurement cuff. The blood pressure waveform at the location of the ascending aorta may then be inferred from the peripheral blood pressure measurement.

Therefore, in the context of such methods, the estimation of the aortic pulse waveform from the peripheral pulse waveform is a critical step. This estimation is a non-trivial task and depends upon the particular vascular dynamics of the patient concerned. Patients having different relevant patient characteristics, e.g. demographics, medical history, medication, will have different vascular tree properties, and accordingly a certain aortic pulse waveform will result in different peripheral pulse waveforms for different people.

SUMMARY OF THE INVENTION

The invention is defined by the independent claims. The dependent claims define advantageous embodiments.

Current methods for transforming a peripheral pressure waveform into a central pressure waveform utilise gender & chronological age to transform the pressure waveform. It is the recognition of the inventors that the accuracy of this transformation (and therefore the accuracy of resulting hemodynamic parameter estimates) can be improved by patient personalisation that reflects in a more fine-grained way characteristics of the patient own vasculature and hemodynamic status.

According to examples in accordance with an aspect of the invention, there is provided a method for estimating an aortic blood pressure pulse waveform, the method comprising:

a) receiving a peripheral arterial pulse waveform for a patient, measured at a peripheral arterial site of the patient;
b) extracting a pre-determined set of one or more waveform features of the peripheral pulse waveform;
c) applying a transformation operation configured to transform the peripheral pulse waveform to an estimate of an aortic pulse waveform for the patient for a same heart pulse as represented by the peripheral pulse waveform;

wherein one or more parameters of the transformation operation are configured in dependence upon the one or more waveform features.

It is proposed in accordance with embodiments of the present invention to use one or more pre-defined arterial waveform features as proxies for personalisation information about the patient's vasculature and to configure the transformation between the peripheral and aortic pulse waveforms accordingly. By using properties of the measured peripheral waveform to configure the transformation operation, this ensures that the personalisation of the transformation reflects true properties of the patient's own personal vasculature. This is in contrast with existing methods which use demographic properties of the patient to configure the transformation operation and which therefore are vulnerable to error when a patient's vasculature is not in accordance with population averages for their given demographic classification.

More particularly, according to at least some embodiments, it is proposed to use waveform feature indicative of, or correlated with, a patient's specific vascular age and/or a patient's current hemodynamic state to configure the transformation from a peripheral pulse waveform to a central pulse waveform.

For avoidance of doubt, a peripheral artery in this context means a non-central artery, e.g. an artery of a limb or extremity, e.g. the brachial artery.

In this disclosure, the reference to an aortic pressure waveform is a reference to a blood pressure waveform at the ascending aorta.

In some embodiments, the transformation operation comprises application of a transfer function. In some embodiments, the aforementioned one or more parameters of the transformation operation include parameters or coefficients of the transfer function.

In some embodiments, the one or more waveform features include one or more features correlated with one or more properties of a vasculature of the patient. For example, this may mean one or more physical properties of the patient vasculature, for example vessel elasticity or compliance, and/or vessel diameter.

In some embodiments, the one or more waveform features include one or more features correlated with a measure of a biological age of a vasculature of the patient.

This might be referred to as patient 'specific vascular age', as contrasted from chronological age. Specific vascular age may provide a more comprehensive understanding of the patient's cardiovascular health, taking into account factors such as, by way of non-limiting example, arterial stiffness, plaque buildup, and overall vascular function.

In some embodiments, the one or more waveform features include one or more features correlated with patient arterial compliance.

Arterial compliance or elasticity is a factor which is related to vascular condition and is in general more indicative of biological vascular age as contrasted from chronological vascular age.

In some embodiments, the one or more waveform features include one or more features indicative of waveform morphology or shape.

In some embodiments, the one or more waveform features include one or more features indicative of one or more transient characteristics of the waveform such as: steepness or gradient of the waveform across at least a portion of the waveform period, rise time, fall time, decay rate, and/or rising rate.

In some embodiments, the one or more waveform features include a feature indicative of a rate of decay of the peripheral pulse waveform during a diastolic decay phase of the pulse waveform. The rate of decay of the pulse waveform has been found to be correlated with arterial compliance, and wherein arterial compliance can be relied upon as a proxy for arterial biological age.

In some embodiments, the aforementioned feature indicative of rate of decay comprises fitting a pre-defined exponential decay function to the diastolic decay phase of the pulse waveform. a time constant of the exponential decay function is a tuneable parameter of the function, and wherein the time constant is used as one of the one or more waveform features. The time constant can for example be taken as a proxy measure for arterial compliance.

In some embodiments, the aforementioned exponential decay function has the form:

$$P(t) = \left(P_{sys} - P_{dia}\right)e^{-\frac{t-t_{sys}}{\tau}} + P_{dia} = \left(P_{sys} - P_{dia}\right)e^{-\frac{t-t_{sys}}{R_{peri}C}} + P_{dia}, \quad for \quad t \in \left[t_{sys}, t_{dia}\right],$$

where $P_{sys} = P(t_{sys})$ [mmHg] is the systolic pressure at the systolic time instant $t_{sys}$ [s], $P_{dia} = P(t_{dia})$ [mmHg] is the diastolic pressure at the diastolic time instant $t_{dia}$ [s], $\tau = R_{peri}C$ [s] is the time constant of the exponential decay, $R_{peri}$ [mmHg.s/cm³] is peripheral vascular resistance and C [cm³/mmHg] is brachial artery compliance.

Physiologically, the time constant $\tau$ is the product of peripheral vascular resistance, $R_{peri}$, and the brachial artery compliance, C. The decay function may be fit to the peripheral waveform to infer a value for the time constant, $\tau$, which can be used as a proxy for vascular compliance. The time constant can be used to configure the transformation operation applied to derive the estimated aortic waveform.

In some embodiments, the method further comprises: obtaining an estimate of pulse wave velocity (PWV) for the subject along an arterial path. The one or more parameters of the transformation operation may be further configured in dependence upon the estimate of pulse wave velocity. The arterial path may be an arterial path which includes the peripheral arterial site at which the peripheral pulse waveform was measured.

In some embodiments, the estimate of PWV can be derived from the measured peripheral pulse waveform.

In some embodiments, one or more of the waveform features may be derived by applying a waveform decomposition operation to the measured peripheral waveform.

In some embodiments, the waveform decomposition operation is a Dynamic Mode Decomposition (DMD).

In some embodiments, the Dynamic Mode Decomposition (DMD) comprises decomposing the peripheral wave function, P(t), into a sum of harmonic components, each harmonic component having a respective radial frequency $\omega_k$ [rad/s], damping coefficient $\delta_k$ [s⁻¹], amplitude $b_k$ [mmHg], and phase $\varphi_k$ [rad], according to the expression:

$$P(t) = \sum_{k=1}^{r} b_k e^{i\varphi_k} e^{-\delta_k t} e^{-i\omega_k t}$$

and wherein the one or more waveform features include at least one of $b_k$, $\varphi_k$, and $\delta_k$ for one or more of the modes, or a feature derived therefrom.

These parameters all provide information about the waveform morphology and vary as a function of vascular compliance. A value of at least one of these parameters for a specific pre-defined one or more modes could be used as the waveform features. In another example, a difference between one or more of these values for two different pre-defined modes could be used as one of the one or more waveform features.

The different DMD frequency components effectively serve as proxies of brachial compliance and pressure wave reflection characteristics within the arterial tree, as well as changes in blood supply generally to different body segments.

In some embodiments, the transformation operation comprises application of a (trained / fitted) regression function, wherein one or more coefficients of the regression function are determined based on the one or more waveform features.

In some embodiments, the method comprises a fitting procedure for fitting the regression function. The fitting procedure may be performed in advance of steps a)-c). The procedure may comprise receiving a plurality of pulse waveform pairs, each pulse waveform pair comprising a peripheral arterial pulse waveform corresponding to a peripheral arterial location and a central arterial pulse waveform corresponding to an aortic location, and the waveforms of each pair corresponding to a same physiological heart pulse. The fitting procedure may further comprise, for each waveform pair, extracting the one or more waveform features from the peripheral waveform of the pair. The procedure may further comprise applying an optimisation procedure to fit a regression function to the data, the regression function for mapping a peripheral waveform to a central waveform as a function of the one or more waveform features.

In some embodiments, the method may further comprise accessing a database storing a plurality of different transfer functions, each configured for transforming a peripheral arterial waveform having a defined set of values for the one or more waveform features into an equivalent aortic arterial waveform, each transfer function indexed in the database according to the said defined set of values of the waveform features, and wherein the configuring the parameters of the transformation operation comprises selecting one of the transform functions from the database according to values of the one or more extracted waveform features of the measured peripheral pulse waveform.

In some embodiments, the method comprises a procedure for compiling the database storing the plurality of different transfer functions. This procedure may comprise: receiving a plurality of pulse waveform pairs, each pulse waveform pair comprising a peripheral arterial pulse waveform corresponding to a peripheral vascular location and a central arterial pulse waveform corresponding to an aortic location, and the waveforms of each pair corresponding to a same physiological heart pulse. The procedure may further comprise, for each waveform pair, extracting the one or more waveform features from the peripheral waveform. The procedure may further comprise, for each waveform pair, determining a transfer function for transforming the peripheral waveform into the aortic waveform. The procedure may further comprise compiling a database recording each determined transfer function, each transfer function indexed in the datastore according to the values of the extracted one or more waveform features.

In some embodiments, each pair of waveforms used in the process of generating each transform function may synthetically be generated, for example using a simulation model.

In some embodiments, the method comprises obtaining a plurality of measurements of the peripheral waveform; and wherein steps b) and c) are performed using an average of the plurality of peripheral waveform measurements.

In some embodiments, the method further comprises computing a standard deviation value. The standard deviation value may be computed by determining a standard deviation of the plurality of peripheral waveform measurements. Alternatively, the standard deviation value may be computed by extracting the one or more waveform features from each of the plurality of measurements of the peripheral waveform and computing a standard deviation of at least one of the waveform features from the plurality of peripheral waveforms. The method may further comprise determining an error measure using the standard deviation value. The method may further comprise determining a confidence interval for the estimated aortic pulse waveform using the error measure.

In some embodiments, the confidence interval may be output to a user interface in combination with the estimated aortic pulse waveform.

In some embodiments, the method further comprises inferring one or more hemodynamic parameters.

In some embodiments, the peripheral arterial site is a site on the brachial artery.

In some embodiments, peripheral blood pressure waveform is measured by a blood pressure measurement apparatus.

In some embodiments, the blood pressure measurement apparatus comprises a pressure applicator for use in applying a variable pressure to the tissue of a body part above a blood vessel. In some embodiments, the blood pressure measurement apparatus comprises a tissue pressure sensor arranged for sensing a pressure between a surface of the user's body part above the blood vessel and the pressure applicator.

In some embodiments, the blood pressure measurement apparatus may comprise a shell structure arranged to extend circumferentially around the body part. The shell structure may have a tubular or broken tubular form. The shell structure may comprise a sheet of material. The sheet of material may curl around the body part when mounted to the body part. The shell structure may include at least one overlapping area where parts of the shell structure circumferentially overlap permitting the shell structure to slide circumferentially to vary an interior tubular diameter of the shell structure. For example, the shell structure may comprise a curled sheet of material which has a circumferential discontinuity such that two circumferential ends of the shell structure are slidable circumferentially over one another.

If the blood pressure measurement apparatus includes a shell structure, in some embodiments, the tissue pressure sensor may comprise a pressure sensor pad situated between the body part and the shell structure during operation, with the pressure applicator disposed radially above the shell structure for example.

The pressure applicator may in some embodiments take the form of an inflatable bladder. However, other options are also possible.

Another aspect of the invention is a processing device comprising one or more processors configured to: a) receive a peripheral arterial pulse waveform for a patient, measured at a peripheral arterial site of the patient; b) extract a pre-determined one or more waveform features of the peripheral pulse waveform; and c) apply a transformation operation configured to transform the peripheral pulse waveform to an estimate of an aortic pulse waveform for the patient for a same heart pulse as represented by the peripheral pulse waveform; wherein one or more parameters of the transformation operation are determined in dependence upon the one or more waveform features.

Another aspect of the invention is a system comprising: the processing device recited above or in accordance with any embodiment described in this disclosure; and a non-invasive pulse waveform measurement means for measuring the peripheral pulse waveform. In some embodiments, the non-invasive pulse waveform measurement means may be a blood pressure measurement apparatus. For example, this may be a blood pressure measurement apparatus in accordance with the example discussed above, or in accordance with any example discussed in this disclosure.

Another aspect of the invention is a method for compiling a database of transfer functions for transforming a peripheral arterial waveform into a central arterial waveform.

The method may comprise:

receiving a plurality of pulse waveform pairs, each pulse waveform pair comprising a peripheral arterial pulse waveform corresponding to a peripheral vascular location and a central arterial pulse waveform corresponding to a central aortic location, and the waveforms of each pair corresponding to a same physiological heart pulse;
extracting one or more pre-defined waveform features from the peripheral waveform;
for each waveform pair, determining a transfer function for transforming the peripheral waveform into the aortic waveform; and
compiling a database recording each determined transfer function, each transfer function indexed in the datastore according to the values of the extracted one or more waveform features.

Another aspect of the invention is a method for fitting a (general) regression transfer function for transforming a peripheral arterial waveform into a central arterial waveform as a function of waveform features extractable from the peripheral waveform.

The method may comprise:

receiving a plurality of pulse waveform pairs, each pulse waveform pair comprising a peripheral arterial pulse waveform corresponding to a peripheral vascular location and a central arterial pulse waveform corresponding to a central aortic location, and the waveforms of each pair corresponding to a same physiological heart pulse;
for each waveform pair, extracting the one or more waveform features from the peripheral waveform of the pair;
applying an optimisation procedure to fit a regression function to the data, the regression function for mapping a peripheral waveform to a central waveform as a function of the one or more waveform features.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 outlines steps of an example method in accordance with one or more embodiments of the invention;
Fig. 2 is a block diagram of an example processing device and system in accordance with one or more embodiments of the invention;
Fig. 3 schematically illustrates a processing flow associated with application of a waveform transformation in accordance with one or more embodiments of the invention;
Fig. 4 schematically illustrates a processing flow associated with application of a waveform transformation in accordance with one or more embodiments of the invention, wherein a transfer function used in the transformation is selected from a database;
Fig. 5 schematically illustrates a processing flow associated with application of a waveform transformation in accordance with one or more embodiments of the invention wherein a single general transfer function is used in the transformation;

Fig. 6 illustrates extraction of a pulse waveform feature indicative according to one or more embodiments of the invention;

Fig. 7 illustrates a method for estimating an aortic blood pressure pulse waveform according to one particular set of embodiments of the invention; and

Fig. 8 illustrates one example blood pressure measurement apparatus suitable for use in measuring a peripheral blood pressure pulse waveform.

## DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a method for inferring a central (e.g. aortic) blood pulse waveform from a peripheral pulse waveform. The method comprises configuring a transformation operation from the peripheral to central waveforms based on one or more waveform features of the measured peripheral pulse waveform.

An aim according to embodiments of the present invention is to enable improved accuracy of non-invasive inference of hemodynamic parameters (such as blood pressure, stroke volume, cardiac output or other cardiac contractility parameters). Certain hemodynamic parameters can be inferred from a measure of an aortic pulse waveform. Embodiments of the present invention provide an improved method for inferring an aortic pulse waveform morphology from a measured peripheral pulse waveform, preferably wherein the measurement of the peripheral pulse waveform is performed non-invasively.

At least one set of embodiments of the invention is based on the concept of tuning the transfer operation used to transform a peripheral pulse to a central pulse based on features or characteristics of the measured peripheral waveform which are indicative of vascular age of patient. For example, vascular compliance changes as a function of age. Therefore, waveform features correlated with vascular compliance may be good candidates for use in configuring the transformation operation.

Embodiments of the invention thus aim to identify optimal transformations or ranges of transformations of peripheral to central blood pressure signals based on current characteristics of the patient.

Embodiments of the invention may provide an estimation of confidence intervals of one or more inferred hemodynamic parameters based on waveform features of the peripherally measured blood pressure waveform.

It is known in the art to measure a blood pressure pulse waveform at a peripheral site and to transform this waveform by means of a transfer function to an aortic blood pressure waveform. It is known to configure the transfer function based on chronological age of the patient. The chronological age is used as a proxy for arterial compliance, based on demographic population data.

Due to the dependency on chronological age, there is a sensitivity of the transformation upon age. If age is incorrect, this may result in a deviation between the predicted aortic pulse waveform output and the actual aortic pulse waveform. The inferred aortic blood pressure can be used to determine hemodynamic parameters such as stroke volume and cardiac output.

However, although chronological age of a patient can be known with precision, the chronological age is not always aligned with biological age. The true vascular age of persons of the same chronological age may be very different, depending on e.g. lifestyle and general health of the patient.

Therefore, it is the recognition of the inventors that the known model for mapping between peripheral and central pulse waveforms might be improved by further optimizing based on factors indicative of true biological vascular age.

One factor which has been found to be correlated with true biological vascular age is arterial stiffness or arterial compliance.

In general terms, arterial stiffness is a measure of a degree of rigidity or flexibility of a subject's arteries. Arterial stiffness is known to increase as a function of biological age. This phenomenon can lead to an increased risk of cardiovascular problems in older age such as high blood pressure, heart attack, and stroke.

Arterial stiffness is mostly dependent on the elasticity of the arterial walls. When arterial walls are elastic, they can expand and contract easily to accommodate the flow of blood. With advancing age, elastic fibres in the arterial walls begin to break down, resulting in reduced elasticity and an increase in arterial stiffness.

Arterial stiffness can be inferred from pulse characteristics of a blood pulse propagating along the arterial tree.

Known methods exist for estimating arterial stiffness.

Arterial stiffness provides a means for characterizing the true "vascular age" of a patient, which differs from the chronological age.

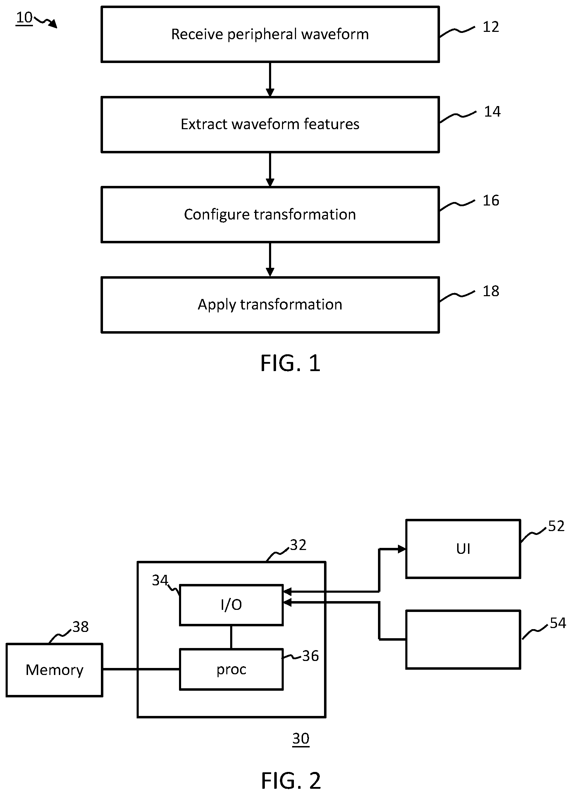

Fig. 1 outlines in block diagram form steps of an example method according to one or more embodiments. The steps will be recited in summary, before being explained further in the form of example embodiments.

Provided is a method 10 for estimating an aortic blood pressure pulse waveform.

The method comprises receiving 12 a peripheral arterial blood pressure pulse waveform for a patient, measured at a peripheral arterial site of the patient, such as the brachial artery.

The method further comprises extracting 14 a pre-determined set of one or more waveform features of the peripheral pulse waveform.

The method further comprises applying 18 a transformation operation configured to transform the peripheral pulse waveform to an estimate of an aortic pulse waveform for the patient for a same heart pulse as represented by the peripheral pulse waveform.

Prior to applying 18 the transformation operation, the method comprises configuring 16 one or more parameters of the transformation operation in dependence upon the one or more waveform features determined in step 14.

As noted above, the method can also be embodied in hardware form, for example in the form of a processing device which is configured to carry out a method in accordance with any example or embodiment described in this document, or in accordance with any claim of this application.

To further aid understanding, Fig. 2 presents a schematic representation of an example processing device 32 configured to execute a method in accordance with one or more embodiments of the invention. The processing device is shown in the context of a system 30 which comprises the processing device. The processing device alone represents an aspect of the invention. The system 30 is another aspect of the invention. The provided system need not comprise all the illustrated hardware components; it may comprise only subset of them.

The processing device 32 comprises one or more processors 36 configured to perform a method in accordance with that outlined above, or in accordance with any embodiment described in this document or any claim of this application. In the illustrated example, the processing device further comprises an input/output 34 or communication interface.

In the illustrated example of Fig. 2, the system 30 further comprises a user interface 52.

In the illustrated example of Fig. 2, the system 30 further comprises a non-invasive pulse waveform measurement means 54 for measuring the peripheral pulse waveform. In some embodiments, the non-invasive pulse waveform measurement means may be a blood pressure measurement apparatus. For example, this may be a blood pressure measurement apparatus in accordance with the example discussed above, or in accordance with any example discussed in this disclosure.

The system 30 may further comprise a memory 38 for storing computer program code (i.e. computer-executable code) which is configured for causing the one or more processors 36 of the processing device 32 to perform the method as outlined above, or in accordance with any embodiment described in this disclosure, or in accordance with any claim.

As mentioned previously, the invention can also be embodied in software form. Thus another aspect of the invention is a computer program product comprising computer program code configured, when run on a processor, to cause the processor to perform a method in accordance with any example or embodiment of the invention described in this document, or in accordance with any claim of this patent application.

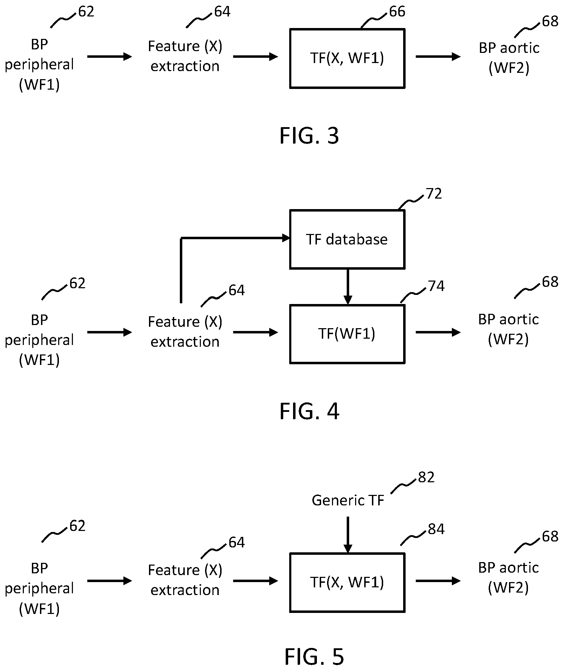

Fig. 3 outlines the basic processing flow according to one or more embodiments. A peripherally measured blood pressure waveform (WF1) 62 is processed to extract one or more waveform features 64, X, of the peripheral waveform 62. A transfer function 66, T, is applied to the peripheral waveform (WF1), wherein parameters of the transfer function are configured using the extracted waveform features, X. The output of the transfer function is a transformed blood pressure waveform (WF2) 68. The transformed blood pressure waveform is an estimate of an equivalent aortic blood pressure waveform which would be expected to be measured at the aorta for the same blood pressure pulse as is represented by the peripheral pulse waveform (WF1). The BP waveform at the aorta may be referred to as a central blood pressure waveform.

The transformation may be represented by the expression:

$$T(X, WF1) \rightarrow WF2$$

where T is the transfer function, X is the one or more waveform features determined from WF1, and WF2 is the estimated aortic waveform function derived from applying WF1.

The expression $T(X, WF1)$ reflects the general concept of configuring the applied transformation, T, in dependence upon the one or more waveform features.

There are at least two general approaches to achieving this. One approach is to determine in advance a plurality of different transfer functions corresponding to (optimized for) use when different particular pre-defined ranges of values for the waveform features are determined from the measured peripheral waveform. For example, a database of different transfer functions is compiled in advance, each derived so as to be optimized for use when certain values of the one or more waveform features are detected. During operation, one of the transfer function is selected by querying the database according to the derived one or more waveform features of the measured peripheral waveform.

This approach is represented schematically in Fig. 4.

As depicted in Fig. 4, the method comprises accessing a database 72 storing a plurality of different transfer functions, T, each configured for transforming a peripheral arterial waveform 62 having a defined set of values, X, for the one or more waveform features 64, into an equivalent aortic arterial waveform 68. Each transfer function is indexed in the database according to the said defined set of values, X, of the waveform features. The previously discussed step of configuring 16 the parameters of the transformation operation in this case comprises selecting one of the transform functions, T, 74 from the database 72 according to values of the one or more extracted waveform features 64, X, of the measured peripheral pulse waveform 62.

In some embodiments, the method of the invention may additionally comprise a procedure for compiling the database 72 storing the plurality of different transfer functions, T. The procedure may be performed prior to steps 12-18 discussed above. The procedure for compiling the database may comprise receiving a plurality of pulse waveform pairs, each pulse waveform pair comprising a peripheral arterial pulse waveform corresponding to a peripheral vascular location and a central arterial pulse waveform corresponding to an aortic location, and the waveforms of each pair corresponding to a same physiological heart pulse. The procedure further comprises, for each pair of waveforms, extracting the one or more waveform features, X, from the peripheral waveform. The procedure further comprises, for each waveform pair, determining a transfer function for transforming the peripheral waveform into the aortic waveform. The procedure further comprises compiling the database 72 recording each determined transfer function, each transfer function indexed in the database according to the values of the extracted one or more waveform features.

In some embodiments, each pair of waveforms used in the process of generating each transform function are synthetically generated. This may comprise using a simulation model.

A second approach to configuring the transformation in dependence upon the extracted waveform features, X, is represented schematically in Fig. 5. In this approach, the method comprises using a generic transfer function 82 which includes one or more coefficients or parameters which take a variable value directly dependent upon values of the extracted one or more waveform features, X. When applying the transfer function, the variable parameters or coefficients are set at values in dependence upon the waveform features, X, of the peripheral BP waveform, and then the configured transfer function 84 is applied to the peripheral waveform.

In this case, the transfer function may be a regression function, wherein one or more coefficients of the regression function are determined based on the one or more waveform features.

The method of the invention may additionally include a process for deriving the generic transfer function. This may be achieved through a regression method, thereby yielding a regression function. This procedure may comprise a fitting procedure for fitting the regression function. The fitting procedure may be performed in advance of steps 12-18 discussed previously. The fitting procedure may comprise receiving a plurality of pulse waveform pairs, each pulse waveform pair comprising a peripheral arterial pulse waveform corresponding to a peripheral arterial location and a central arterial pulse waveform corresponding to an aortic location, and the waveforms of each pair corresponding to a same physiological heart pulse. The fitting procedure may further comprise, for each waveform pair, extracting the one or more waveform features from the peripheral waveform of the pair. The procedure may further comprise applying an optimisation procedure to fit a regression function to the data, the regression function for mapping a peripheral waveform to a central waveform as a function of the one or more waveform features.

In some embodiments, the regression function may be trained to additionally depend upon one or more physiological parameters, such as pulse wave velocity, pulse arrival time, or pulse transit time between two arterial sites.

In some embodiments, the regression function may be trained to additionally depend upon one or more variables related to patient characteristics. These may include for instance gender, chronological age, height, and weight.

As discussed above, embodiments of the invention involve extracting one or more waveform features from the peripherally measured blood pressure waveform. There are a number of options with regards to the particular waveform features which are extracted. The options in this regard will now be discussed in more detail.

In accordance with at least one set of embodiments, the waveform features which are extracted may include one or more waveform features which are correlated with one or more properties of a vasculature of the patient.

In an advantageous set of embodiments, the one or more waveform features may include one or more features correlated with a measure of a biological age of a vasculature of the patient.

By way of example, according to at least one advantageous set of embodiments, the one or more waveform feature may include one or more features correlated with patient arterial compliance.

There are different ways to target proxies of arterial compliance. Two main approaches will be outlined below. One approach is based on extracting one or more inherent properties or features of the measured peripheral waveform itself. By this is meant for example one or more features indicative of a morphology or shape of the measured peripheral waveform.

In particular, it has been found that features indicative of one or more transient characteristics of the waveform such as: steepness or gradient of the waveform across at least a portion of the waveform period, rise time, fall time, decay rate, and/or rising rate are effectively proxies or indicators or arterial compliance, since the rate at which pressure rises or falls during a blood pulse is a function of the elasticity of the artery walls at the point of measurement.

By way of one particular example, the one or more waveform features extracted from the peripherally measured waveform may include a feature indicative of a rate of decay of the peripheral pulse waveform during a diastolic decay phase of the pulse waveform. There are different ways of evaluating a measure of a rate of decay of a wavefunction, as the skilled person will be aware.

According to one particular approach, discussed in more detail below, the determining the feature indicative of rate of decay may be performed by fitting a pre-defined exponential decay function to the diastolic decay phase of the pulse waveform. For example, a time constant of the exponential decay function may be a tuneable parameter of the function, and wherein the time constant is used as one of the one or more waveform features.

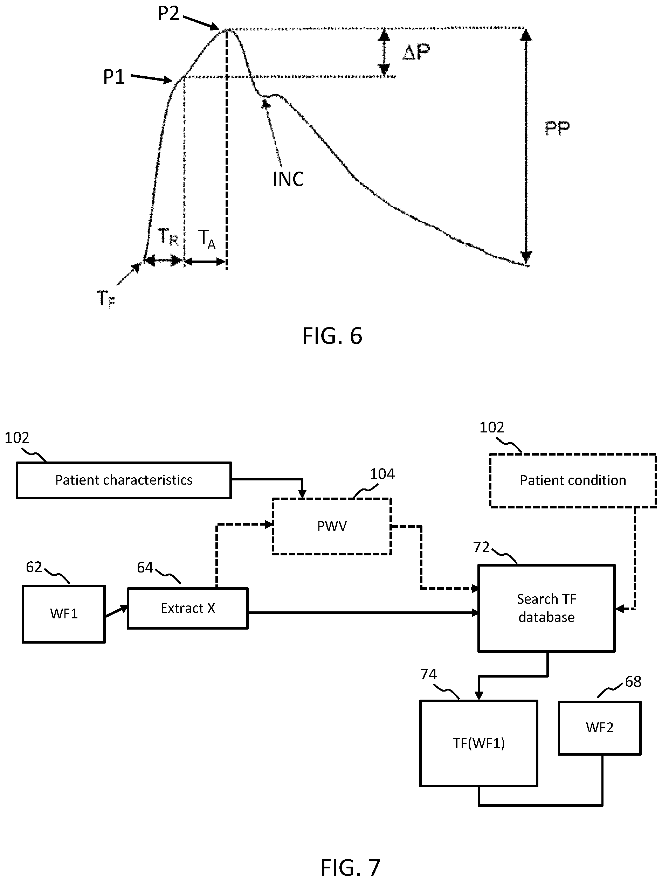

By way of illustration, Fig. 6 schematically illustrates an example blood pressure pulse waveform measured at a peripheral arterial site. Here, $T_F$ represents the foot of the pulse wave, P1 represents the first systolic peak of the pulse wave, P2 represents the second systolic peak of the pulse wave, $\Delta P$ represents the augmentation pressure, PP represents the pulse pressure, INC represents the incisura (dicrotic notch) of the pulse wave, $T_R$ is the time difference between the foot of the wave and the first systolic peak, P1, and $T_A$ is the time difference between the first systolic peak, P1, and the second systolic peak, P2.

The diastolic decay phase of the pulse waveform may be defined as the portion of the wavefunction following the second systolic peak, P2, or the portion of the wavefunction following the dicrotic notch, INC. This diastolic decay phase of the pulse wavefunction may be represented by an exponential decay function.

According to one set of examples, the exponential decay function has the form:

$$P(t) = \left(P_{sys} - P_{dia}\right)e^{-\frac{t-t_{sys}}{\tau}} + P_{dia} = \left(P_{sys} - P_{dia}\right)e^{-\frac{t-t_{sys}}{R_{peri}C}} + P_{dia}, \quad for \quad t \in \left[t_{sys}, t_{dia}\right],$$

where $P_{sys} = P(t_{sys})$ [mmHg] is the systolic pressure at the systolic time instant $t_{sys}$ [s], where $t_{sys}$ corresponds to the time of P2, $P_{dia} = P(t_{dia})$ [mmHg] is the diastolic pressure at the diastolic time instant $t_{dia}$ [s], where $t_{dia}$ corresponds to the time of $T_F$, $\tau = R_{peri}C$ [s] is the time constant of the exponential decay, $R_{peri}$ [mmHg.s/cm$^3$] is peripheral vascular resistance and $C$ [cm$^3$/mmHg] is brachial artery compliance.

The exponential decay function, P(t) may be fit to the diastolic decay phase of the measured peripheral pulse waveform. To achieve this, the time constant, $\tau$, of the exponential decay function may be used as a tuneable parameter of the decay function, P(t), and a fitting procedure is done wherein $\tau$ is adjusted to optimise fit of P(t) with the diastolic decay phase of the measured peripheral pulse waveform. This may be done for example through a regression fitting operation. Once P(t) has been fitted to the diastolic decay phase of the peripheral pulse waveform, the resulting value of the time constant, $\tau$, may be used as one of the previously discussed one or more waveform features for use in configuring the transformation operation.

Physiologically, the time constant $\tau$ is the product of peripheral vascular resistance, $R_{peri}$, and the brachial artery compliance, C. As taught above, the decay function is fit to the peripheral waveform to infer a value for the time constant, $\tau$. The time constant, $\tau$, effectively represents a proxy for vascular compliance. The time constant can be used to configure the transformation operation applied to derive the estimated aortic waveform.

The above represents one way of targeting waveform features which are proxies are arterial compliance. Another approach will now be discussed.

According to a further approach, a measure of pulse wave velocity, or a correlate thereof, may be derived using the peripheral pulse wave, and this may be used as one of the waveform features used for configuring the transformation operation. Pulse wave velocity, PWV, is known to be correlated with arterial compliance.

To state this more explicitly, according to some embodiments, the method may further comprise: obtaining an estimate of pulse wave velocity (PWV) for the subject along an arterial path, and wherein the previously discussed one or more parameters of the transformation operation are further configured in dependence upon the estimate of pulse wave velocity. The said arterial path may be an arterial path which includes the peripheral arterial site at which the peripheral pulse waveform was measured.

One way of determining an estimate of pulse wave velocity is by analysis of intrinsic features of the measured peripheral pulse waveform. This approach will now be discussed, with reference again to Fig. 6.

With reference to Fig. 6, the first systolic peak (P1) represents the initial wave of blood pressure created directly by the contraction of the left ventricle of the heart. When the heart contracts during systole, it sends a wave of blood through the arteries. The peak of this initial wave, or upstroke, seen on the pulse waveform, is the first systolic peak.

The second systolic peak (P2) occurs after the first systolic peak (P1) and is a result of the reflection of the pulse wave from the peripheral arteries back towards the heart. When the initial wave of blood pressure travels through the arteries, it eventually encounters points of resistance or impedance changes (such as bifurcations or areas of arterial stiffness). This causes a part of the wave to be reflected back towards the heart. The second systolic peak on the waveform represents this reflected wave. The timing and amplitude of this second peak can vary based on factors such as arterial stiffness and the distance to the reflection sites.

The time difference between the foot of the wave (at time $T_F$) and first systolic peak, P1, denoted as $T_R$, represents the time it takes for the pulse wave to travel from the heart to a point of reflection (often in the peripheral arteries) and back to the point of measurement.

The pulse pressure (PP) is the pressure change between the first systolic peak (P1) and the second systolic peak (P2).

The augmentation pressure ($\Delta$P) is the difference in pressure between the first systolic peak (P1) and the second systolic peak (P2) of the pulse waveform. It represents the additional pressure in the arterial system due to reflected waves returning to the heart.

The time between the first systolic peak, P1, and the second systolic peak, P2, is denoted at $T_A$.

From the augmentation pressure ($\Delta$P), an augmentation index (Aix) can be derived which represents the augmentation pressure $\Delta$P divided by the pulse pressure, PP. The augmentation index can be used as an indicator of arterial stiffness and wave reflection in the arterial tree. A higher augmentation index suggests increased arterial stiffness and enhanced wave reflection.

Using the measured peripheral pulse wave, an estimate of PWV can be determined based on fiducial points of the pulse waveform. In particular, a time difference, $T_A$, between the first systolic peak and the second systolic peak can be used as a proxy for transit time of the pulse wave from the heart. For example, a pre-determined transfer function can be retrieved and applied to this time difference, $T_A$, to transform it to an estimated transit time from the heart to the measurement site of the peripheral pulse waveform. An estimate may further be made of the arterial path length from the heart to the peripheral measurement location for example using population-based lookup tables which map from height to estimated arterial path length to a defined peripheral measurement location (e.g. finger).

Additionally or alternatively to the above, when additional measurements are available for the patient, such as ECG or PPG, measures of pulse arrival time, PAT, and/or pulse transit time, PTT, can be derived, each of these also representing measures correlated with arterial compliance. Furthermore, from either PAT or PTT, a measure of PWV can be derived.

Pulse arrival time (PAT) can for instance be determined as the time difference between the time instant of the R-peak in an ECG signal and the time of the onset of a pulse at a downstream measurement location in the vasculature. For example, it could be measured as the time between the R-peak and the time of onset of the peripheral pulse waveform measured using the blood pressure measurement apparatus, i.e. $T_F$ in the example pulse wave of Fig. 6. Additionally or alternatively, if a PPG sensor is available, the PPG sensor can be used to detect onset of the pulse wave at a peripheral site, such as the finger, and this used to determine a measure of PAT.

From PAT, a measure of PWV can be derived by taking into account the heart's pre-ejection period (PEP), as PAT = PEP + PTT. The PEP is subtracted from the measure of PAT to thereby yield a value for PTT. An estimate is made of the path length travelled by the pulse between the aortic inlet and the measurement site, for example based on measurement or based on population values using demographic information of the patient.

A measure of pulse wave velocity may then be derived as PWV = Path Length / PTT.

Another way to derive a measure of PWV is to measure the time taken for a pulse wave to travel from a first peripheral arterial site to a second peripheral arterial site, the second site being downstream from the first along a common arterial path. An estimate can be made of the path length between the first and second arterial sites, e.g. by physical measurement of the distance, and a measure of PWV derived as the Path Length / Travel time between site 1 and site 2. The onset of the pulse at site 1 and site 2 could be measured using a blood pressure measurement apparatus at one site and a PPG sensor at the other site, or by using two PPG sensors. In some embodiments, the two sites may be two sites along the length of a blood pressure measurement cuff, and wherein a pair of PPG sensors are integrated in the cuff, spaced along the dimension running parallel with the body part when the cuff is worn.

Above have been described a number of examples of waveform features which may be used to configure the transformation operation, T. Most of these have involved direct extraction of features related to the morphology or shape of the pulse waveform.

Additionally or alternatively to those discussed above, in some embodiments, waveform features may be extracted by performing a decomposition of the waveform and extracting one or more decomposition components.

In other words, one or more of the waveform features may be derived by applying a waveform decomposition operation to the measured peripheral waveform.

One advantageous approach identified by the examiners is the use of a dynamic mode decomposition.

A Dynamic Mode Decomposition (DMD) comprises decomposing the peripheral waveform P(t) into a sum of harmonic components, each harmonic component having a respective radial frequency $\omega_k$ [rad/s], damping coefficient $\delta_k$

[s$^{-1}$], amplitude $b_k$ [mmHg], and phase $\varphi_k$ [rad], according to the expression:

$$P(t) = \sum_{k=1}^{r} b_k e^{i\varphi_k} e^{-\delta_k t} e^{-i\omega_k t}$$

According to one set of embodiments, the one or more waveform features used to configure the transformation operation may be derived based on values for at least one of $b_k$, $\varphi_k$, and $\delta_k$ for one or more of the modes. These parameters all provide information about the waveform morphology and vary as a function of vascular compliance. A value of at least one of these parameters for a specific pre-defined one or more modes could be used as the waveform features. In another example, a difference between one or more of these values for two different pre-defined modes could be used as one of the one or more waveform features.

By way of example, the phase difference between the first and second (or higher) mode may provide information about waveform reflection and the resulting waveform shape and this reflection/ resulting shape depends on the vascular age/ condition. Thus one waveform feature used to configure the transformation operation may be given by $\varphi_b - \varphi_a$, where $a>b$ and preferably wherein b = 1. For example $a$ may be 2.

A physiological understanding of the DMD components may be given as follows. Centralization is a patient condition in which blood supply to peripheral body segments (such as arms, legs) is reduced by an increased peripheral resistance. This occurs so as to keep the essential body part (the "core") perfused.

Occurrence of this condition can be observed in the amplitudes or amplitude ratios, and in the phase relations of the different DMD frequency components serving as proxy of the brachial compliance.

In the preceding descriptions, a single peripheral pulse waveform measurement has been assumed. However, to improve robustness of measurement, it may be advantageous to measure a plurality of peripheral pulse waveforms and either derive from these a representative waveform which may then be transformed to an equivalent aortic waveform, or derive from these a representative set of values of the one or more waveform features.

For example, in some embodiments, the method may comprise: obtaining a plurality of measurements of the peripheral waveform; and wherein method steps 14, 16 and 18 are performed using an average of the plurality of peripheral waveform measurements.

The plurality of different peripheral pulse measurements might be obtained in a sequence, one after the other. In some examples, a position of the patient might be adjusted between each measurement to obtain a diverse set of samples. For example, events such as passive leg raise or breath hold can be performed and the different measures of the pulse waveform acquired before, during and/or after these events.

By way of one specific example, values for $b_k$ and $\varphi_k$ of one or more defined DMD components of each of the plurality of peripheral pulse waveforms may be extracted and these may be ensemble averaged to yield ensemble average values for $b_k$ and $\varphi_k$ of the defined DMD components.

In addition, in some embodiments, the method may further comprise computing a confidence interval or confidence band for the aortic waveform transformed from the peripheral waveform. This can be realised for example by computing a standard deviation value, where the standard deviation value is computed based on either (1) determining a standard deviation of the plurality of peripheral waveform measurements, or (2) extracting the one or more waveform features from each of the plurality of measurements of the peripheral waveform and computing a standard deviation of at least one of the waveform features from the plurality of peripheral waveforms. The method may further comprise determining an error measure using the standard deviation value; and determining a confidence interval for the estimated aortic pulse waveform using the error measure.

The confidence interval may be presented to the user via the user interface. It may be presented to the user via the user interface in combination with the derived aortic waveform.

As discussed above, the method may comprise determining one or more hemodynamic parameters from the aortic waveform derived by applying the transformation operation to the measured peripheral waveform.

Using the confidence interval derived for the aortic pulse waveform, a corresponding confidence interval or confidence band may be derived for each of the inferred hemodynamic parameters.

The confidence interval(s) for the one or more hemodynamic parameters may be presented to the user via output to the user interface, for example presented in combination with the corresponding hemodynamic parameters. A warning may be presented in the event that a confidence interval is indicative of low certainty of the hemodynamic parameter estimation, for example is the confidence interval width exceeds a certain amount.

Furthermore, in some embodiments, a threshold uncertainty in an estimated hemodynamic parameter may also trigger assessment as to whether the patient is currently subject to a pre-defined haemodynamic event.

With regards to the computation of a confidence interval for the aortic waveform, this may be done in the following way.

As mentioned previously, a plurality of peripheral blood pressure pulse measurements may be obtained, from the same measurement site. For example they may be obtained in a sequence, one after the other.

For each measurement of the peripheral waveform, the transfer function, T, is configured based on the one or more relevant waveform features of the peripheral waveform, and the transfer function is then applied to the peripheral waveform, to thereby yield a plurality of predicted aortic waveforms.

Once the plurality of aortic waveforms are derived, statistical analysis is performed to estimate a confidence interval. The steps are as follows. First, a mean aortic waveform is determined by computing the mean of the set of predicted aortic waveforms. This mean prediction represents the central tendency of the predictions. Next, a standard deviation (SD) or a standard error (SE) is computed of the set of aortic waveforms. The standard error is the standard deviation divided by the square root of the sample size (SE = SD/sqrt(n)). Computation of the standard deviation or standard error is described in more detail below.

The mean and the standard error can be used to calculate the confidence interval. For a 95% confidence interval, the formula is: CI = Mean $\pm$ (1.96 * SE) where 1.96 is the Z-score for a 95% confidence level.

The confidence interval gives a range within which the true aortic pulse waveform is expected to fall, with a certain level of confidence (e.g., 95%). A narrower confidence interval implies higher precision in the predictions, while a wider interval indicates greater uncertainty.

With regards to computing a standard deviation of the set of predicted aortic waveforms, this may be done as follow. Here, the set of aortic waveforms may be represented by the waveform function $A_n(x)$, where x indexes the samples in each waveform, and n indexes the waveforms and runs from 1 to N.

A first step is to compute a mean of the N aortic waveforms. This results in a mean aortic waveform function, $\langle A_n(x) \rangle$. This is done by averaging the values of each sample point across all waveforms. If $A_n(x)$ represents the value of the nth waveform at sample point x, the mean $\langle A_n(x) \rangle$ at each sample point x is calculated as:

$$\langle A_n(x) \rangle = \frac{1}{N} \sum_{n=1}^{N} A_n(x)$$

Next, to compute the standard deviation, SDV $\{A_n(x)\}$ at each sample point x, the following expression can be used:

$$SDV\{A_n(x)\} = \sqrt{\frac{1}{N} \sum_{n=1}^{N} (A_n(x) - \langle A_n(x) \rangle)^2}$$

In this formula, $(A_n(x) - \langle A_n(x) \rangle)^2$ calculates the squared difference between each waveform value at point x and the mean value at that point.

An example implementation of the method according to a particular set of embodiments will now be described by way of illustration of the above-summarized concept of the invention, with reference to Fig. 7. It will be appreciated that not all features of this particular set of embodiments are essential to the inventive concept, and are described to aid understanding and to provide an example to illustrate the inventive concepts.

Fig. 7 outlines a block diagram of an example method for obtaining a personalized transfer function from brachial to aortic pressure.

A peripheral blood pressure waveform (WF 1) 62 is measured for the patient. One or more waveform features, X, are extracted 64 from the peripheral waveform 62. These waveform features may be features correlated with arterial compliance of the patient.

Optionally, one or more patient characteristics 102 may be combined with the waveform features, X, to derive a measure of PWV 104, using the method discussed previously.

The waveform features, optionally in combination with the PWV 104, are used to query a transfer function database 72 to identify an optimum transfer function or range of transfer functions for use in converting the measured peripheral waveform WF1 to the aortic waveform WF2.

The transfer function is applied 74 to the peripheral waveform to yield an estimated aortic waveform 68 or estimated range of aortic blood pressure waveforms.

In some embodiments, an estimate of one or more hemodynamic parameters is derived using the aortic waveform, e.g. stroke volume (SV) and/or cardiac output (CO).

In case multiple aortic BP waveforms are determined following a confidence interval approach, as discussed above, multiple SV/CO estimations can accordingly be realized, also with associated confidence intervals.

Reference is made to the following paper: Aubert, X. L., Muehlsteff, J., & Zhu, C. (2007, August). Relationships between blood pressure and systolic time-intervals: a lumped-model simulation study. In 2007 29th Annual International Conference of the IEEE Engineering in Medicine and Biology Society (pp. 1707-1710). IEEE. This describes an example method for computing various hemodynamic parameters from the aortic pulse waveform.

As discussed above, some embodiments involve determining a database of transfer functions, each indexed in the database according to the values of the extracted one or more waveform features, and wherein this involves a stop of obtaining a plurality of pulse waveform pairs, each pulse waveform pair comprising a peripheral arterial pulse waveform corresponding to a peripheral vascular location and a central arterial pulse waveform corresponding to an aortic location, and the waveforms of each pair corresponding to a same physiological heart pulse.

Likewise, some embodiments involve fitting a regression function for use as a transfer function, and wherein the data used to fit the regression function includes a plurality of pulse waveform pairs, each pulse waveform pair comprising a peripheral arterial pulse waveform corresponding to a peripheral vascular location and a central arterial pulse waveform corresponding to an aortic location, and the waveforms of each pair corresponding to a same physiological heart pulse.

In either case, to obtain the plurality pulse waveform pairs, different options are possible.

In one set of embodiments, the plurality of pulse waveform pairs may be obtained from real historical patient data. Historical patient data can e.g. be hospital patient data that has been acquired over time, which represents a local specific population of that hospital.

In another set of embodiments, the plurality of pulse waveform pairs may be obtained using a simulation model. A simulation model can e.g. be used to generate data to represent an artificial patient population having appropriate characteristics, e.g. for the hospital department of interest.

A simulation model may comprise a blood propagation model of the arterial tree.

The propagation of a blood pressure waveform throughout the arterial tree can be modelled using 1D blood flow models. Reference is made for example to the following paper: J. Alastruey, K. H. Parker, and S. J. Sherwin, "Arterial pulse wave haemodynamics," BHR Group - 11th Int. Conf. Press. Surges, pp. 401-442, 2012. This details an example 1D model of propagation of a blood pressure waveform.

Various 1D models described in literature have been shown to reproduce both physiological waveform morphology and physiological phenomena such as pulse pressure amplification and pulse wave propagation. By way of example, reference is made to the following papers which describe examples of such 1D models:
J. P. Mynard and J. J. Smolich, "One-Dimensional Haemodynamic Modeling and Wave Dynamics in the Entire Adult Circulation," Ann. Biomed. Eng., vol. 43, no. 6, pp. 1443-1460, Jun. 2015, doi: 10.1007/s10439-015-1313-8.

P. Reymond, F. Merenda, F. Perren, D. Rüfenacht, and N. Stergiopulos, "Validation of a one-dimensional model of the systemic arterial tree," Am. J. Physiol.-Heart Circ. Physiol., vol. 297, no. 1, pp. H208-H222, 2009, doi: 10.1152/ajpheart.00037.2009.

J. Alastruey, N. Xiao, H. Fok, T. Schaeffter, and C. A. Figueroa, "On the impact of modelling assumptions in multi-scale, subject-specific models of aortic haemodynamics," J. R. Soc. Interface, vol. 13, no. 119, p. 20160073, Jun. 2016, doi: 10.1098/rsif.2016.0073.

These models can be used to model pairs of peripheral and central pulse waveforms corresponding to a same heart pulse event. The 1D model may be for example parameterised by parameters such as arterial length, arterial diameter, arterial stiffness. By varying these parameters, virtual populations can be created and pairs of peripheral and central pulse waveforms generated for each virtual population.

By simulating a wide range and combination of these parameters, these virtual populations can provide further insight into physiological variations than clinical studies.

By way of example, recent examples of such virtual populations are detailed in the following papers:
M. Willemet, P. Chowienczyk, and J. Alastruey, "A database of virtual healthy subjects to assess the accuracy of foot-to-foot pulse wave velocities for estimation of aortic stiffness," Am. J. Physiol.-Heart Circ. Physiol., vol. 309, no. 4, pp. H663-H675, 2015.

P. H. Charlton, J. Mariscal Harana, S. Vennin, Y. Li, P. Chowienczyk, and J. Alastruey, "Pulse Wave Database (PWDB): A database of arterial pulse waves representative of healthy adults," 2019.

By way of example, to create a virtual population, a chronological age may be translated into measures of arterial stiffness and arterial diameter, which in combination with heart rate and cardiac output can be to simulate a population of different waveforms from different patients. In each virtual patient both the chronological and vascular age are known, enabling further insight into the correlation with waveform morphology.

As discussed above, in some embodiments, the peripheral blood pressure waveform measurement is obtained using a blood pressure measurement apparatus. This may for example be a non-invasive blood pressure (NIBP) measurement cuff.

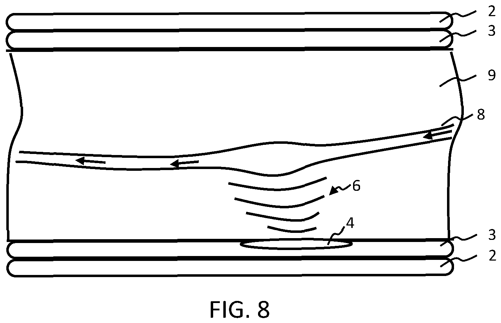

One example blood pressure measurement apparatus suitable for use in accordance with one particular set of embodiments is outlined in Fig. 8. This provides a cuff-based hemodynamic parameter measurement apparatus. The apparatus can be used to detect blood pressure and/or other hemodynamic parameter measurements such as cardiac output or stroke volume.

The illustration shows the cuff applied to the upper arm 9 of a patient. An artery 8 of the patient is schematically shown. The apparatus has a design which differs from the most standard cuff-based blood pressure measurement apparatuses in that it includes a dedicated tissue pressure sensor 4 which the cuff acts to hold against the tissue of the skin when the cuff is inflated. The cuff includes a pneumatic actuator in the form of an inflatable bladder 2 for use in changing a pressure applied to the body part 9 by the cuff. The tissue pressure sensor 4 is arranged for sensing a pressure between a surface of the user's body part and the cuff. Independently of the tissue pressure sensor, one or more operational parameters of the pneumatic actuator can be sampled. For example, a pressure inside the inflatable bladder can be sensed. An actuator activity signal indicative of a pumping power level or pumping rate of a pump of the pneumatic actuator can also be sampled.

Standard blood pressure measurement cuffs use the bladder air pressure as the sole measurement signal for sensing blood pressure. By additionally utilizing a tissue pressure sensor 4, the apparatus shown in Fig. 8 can achieve superior quality results and also enables the measurement of advanced hemodynamic parameters such as stroke volume, cardiac output and fluid responsiveness in addition to blood pressure. In particular, in standard air blood pressure cuffs, dampening air is assumed to destroy > 90% of the amplitude and contour of a tissue pressure pulse wave. By contrast, by using a dedicated tissue pressure sensor, the design illustrated in Fig. 8 allows recording high-fidelity (HiFi) artery blood pressure and pulse waveform.

The tissue pressure sensor 4 may be implemented as, e.g. a fluid-filled bag or pad. This may therefore provide a conforming layer of fluid between the cuff bladder 2 and the surface of the user's body. In this way, the integrated pneumatic actuator enables hydraulic coupling of the tissue pressure sensor 4 to the upper arm tissue. When blood pulses in the artery, this results in a pressure wave 6 which can be detected by the tissue pressure sensor 4. The tissue pressure sensor 4 can be connected to a pressure transducer via a fluid-filled tube/line. The pressure transducer converts the pressure in the fluid into an electrical signal.

The operating principle is based on coupling of the pressure sensor to the body part (e.g. arm) in order to transcutaneously record tissue pressure pulse waves that result from arterial pulsation (e.g. brachial artery). In similar fashion to a conventional upper arm air blood pressure cuff, the cuff compresses the upper arm using an integrated pneumatic actuator with rising clamping pressure. However, the actual compression may be achieved via the narrowing diameter of a rigid circumferential shell.

In the example illustrated in Fig. 8, the cuff includes a shell portion 3, wherein the shell portion is arranged so as to be located between the pneumatic actuator 2 and the body part when the cuff is worn on the body part, and arranged to surround the body part when the cuff is worn. The tissue pressure sensor 4 is arranged so as to be positioned between the shell 3 and the body part when the device is worn on the body part. The shell structure may be relatively stiff. Consequently, the measurement accuracy can be improved, since the tissue pressure measured by tissue pressure sensor 4 is measured against a relatively stiff support, which prevents dampening of the amplitude and shape of the signal. In particular, if a tissue pressure sensor unit 4 is located at least partially between the shell portion 3 and the body part, a high accuracy of the signals measured by the tissue pressure sensor unit can be achieved. With such a configuration of the tissue pressure sensor unit, the shell structure does not absorb or attenuate the arterial pressure signal.

The shell portion 3 may include overlapping sections, and wherein the overlapping sections of the shell portion may move or slide relatively to each other, thereby reducing the diameter of the shell portion responsive to increasing applied pressure by the pneumatic actuator.

If the tissue pressure sensor uses a fluid-based sensing mechanism, as described above, then an initial calibration may be needed in which the pressure transducer, to which the fluid filled pad 4 of the pressure sensor is fluidically coupled, is exposed to atmospheric pressure prior to beginning the measurement. A calibration or correction to account for hydrostatic pressure differences may also be performed.

The design described above permits non-invasive hemodynamic monitoring and enables measurement of blood pressure, as well as cardiac output and other hemodynamic parameters.

Embodiments of the invention described above employ a processing device. The processing device may in general comprise a single processor or a plurality of processors. It may be located in a single containing device, structure or unit, or it may be distributed between a plurality of different devices, structures or units. Reference therefore to the processing device being adapted or configured to perform a particular step or task may correspond to that step or task being performed by any one or more of a plurality of processing components, either alone or in combination. The skilled person will understand how such a distributed processing device can be implemented. The processing device includes a communication module or input/output for receiving data and outputting data to further components.

The one or more processors of the processing device can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

A computer program may be stored/distributed on a suitable medium, such as an optical storage medium or a solid-state medium supplied together with or as part of other hardware, but may also be distributed in other forms, such as via the Internet or other wired or wireless telecommunication systems.

## Claims

1.   A method for estimating an aortic blood pressure pulse waveform, the method comprising:

   a) receiving a peripheral arterial pulse waveform for a patient, measured at a peripheral arterial site of the patient;
   b) extracting a pre-determined set of one or more waveform features of the peripheral pulse waveform;
   c) applying a transformation operation configured to transform the peripheral pulse waveform to an estimate of an aortic pulse waveform for the patient for a same heart pulse as represented by the peripheral pulse waveform;

   wherein one or more parameters of the transformation operation are configured in dependence upon the one or more waveform features.

2.   The method of claim 1, wherein the one or more waveform features include one or more features correlated with patient arterial compliance.

3.   The method of any preceding claim,

   wherein the one or more waveform features include one or more features indicative of waveform morphology or shape; and/or
   wherein the one or more waveform features include one or more features indicative of one or more transient characteristics of the waveform such as: steepness or gradient of the waveform across at least a portion of the waveform period, rise time, fall time, decay rate, and/or rising rate.

4.   The method of any preceding claim, wherein the one or more waveform features include a feature indicative of a rate of decay of the peripheral pulse waveform during a diastolic decay phase of the pulse waveform.

5.   The method of claim 4, wherein determining the feature indicative of rate of decay comprises fitting a pre-defined exponential decay function to the diastolic decay phase of the pulse waveform; and optionally wherein a time constant of the exponential decay function is a tuneable parameter of the function, and wherein the time constant is used as one of the one or more waveform features.

6.   The method of any preceding claim, wherein one or more of the waveform features are derived by applying a waveform decomposition operation to the measured peripheral waveform.

7.   The method of claim 6, wherein the waveform decomposition operation is a dynamic mode decomposition.

8.   The method of claim 7, wherein the Dynamic Mode Decomposition (DMD) comprises decomposing the peripheral wave function P(t) into a sum of harmonic components, each harmonic component having a respective radial frequency $\omega_k$ [rad/s], damping coefficient $\delta_k$ [s$^{-1}$], amplitude $b_k$ [mmHg], and phase $\varphi_k$ [rad], according to the expression:

$$P(t) = \sum_{k=1}^{r} b_k e^{i\varphi_k} e^{-\delta_k t} e^{-i\omega_k t}$$

and wherein the one or more waveform features include at least one of $b_k$, $\varphi_k$, and $\delta_k$ for one or more of the modes, or a feature derived therefrom.

9. The method of any preceding claim, wherein the transformation operation comprises application of a regression function, wherein one or more coefficients of the regression function are determined based on the one or more waveform features; and optionally wherein the method comprises a fitting procedure for fitting the regression function, the fitting procedure performed in advance of steps a)-c).

10. The method of any of claims 1-9,

wherein the method comprises accessing a database storing a plurality of different transfer functions, each configured for transforming a peripheral arterial waveform having a defined set of values for the one or more waveform features into an equivalent aortic arterial waveform, each transfer function indexed in the database according to the said defined set of values of the waveform features, and

wherein the configuring the parameters of the transformation operation comprises selecting one of the transform functions from the database according to values of the one or more extracted waveform features of the measured peripheral pulse waveform.

11. The method of claim 10, wherein the method comprises a procedure for compiling the database storing the plurality of different transfer functions, the method comprising:

receiving a plurality of pulse waveform pairs, each pulse waveform pair comprising a peripheral arterial pulse waveform corresponding to a peripheral vascular location and a central arterial pulse waveform corresponding to an aortic location, and the waveforms of each pair corresponding to a same physiological heart pulse;

for each waveform pair, extracting the one or more waveform features from the peripheral waveform;

for each waveform pair, determining a transfer function for transforming the peripheral waveform into the aortic waveform; and

compiling a database recording each determined transfer function, each transfer function indexed in the database according to the values of the extracted one or more waveform features.

12. The method of any preceding claim,

wherein the method comprises obtaining a plurality of measurements of the peripheral waveform; and

wherein steps b) and c) are performed using an average of the plurality of peripheral waveform measurements.

13. The method of claim 12, wherein the method further comprises:

computing a standard deviation value by:

determining a standard deviation of the plurality of peripheral waveform measurements; or

extracting the one or more waveform features from each of the plurality of measurements of the peripheral waveform and computing a standard deviation of at least one of the waveform features from the plurality of peripheral waveforms;

determining an error measure using the standard deviation value; and

determining a confidence interval for the estimated aortic pulse waveform using the error measure.

14. A processing device comprising one or more processors configured to:

a) receive a peripheral arterial pulse waveform for a patient, measured at a peripheral arterial site of the patient;

b) extract a pre-determined one or more waveform features of the peripheral pulse waveform; and

c) apply a transformation operation configured to transform the peripheral pulse waveform to an estimate of an aortic pulse waveform for the patient for a same heart pulse as represented by the peripheral pulse waveform;

wherein one or more parameters of the transformation operation are determined in dependence upon the one or more waveform features.

**15.** A system comprising:

the processing device of claim 14; and
a non-invasive pulse waveform measurement means for measuring the peripheral pulse waveform.

FIG. 1

FIG. 2

62

BP
peripheral
(WF1)

64

Feature (X)
extraction

66

TF(X, WF1)

68

BP aortic
(WF2)

FIG. 3

72

TF database

62

BP
peripheral
(WF1)

64

Feature (X)
extraction

74

TF(WF1)

68

BP aortic
(WF2)

FIG. 4

82

Generic TF

62

BP
peripheral
(WF1)

64

Feature (X)
extraction

84

TF(X, WF1)

68

BP aortic
(WF2)

FIG. 5

FIG. 6

FIG. 7

FIG. 8

| | Europäisches Patentamt European Patent Office Office européen des brevets | **EUROPEAN SEARCH REPORT** | **Application Number** EP 24 17 1963 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2019/298191 A1 (MUKKAMALA RAMAKRISHNA [US] ET AL) 3 October 2019 (2019-10-03) * paragraphs [0029], [0031] - [0033], [0035], [0038], [0042] - [0043], [0046] - paragraphs [0061] - [0062]; claims 1,2,5 * | 1-15 | INV. A61B5/021 A61B5/00 |
| A | DU SHUO ET AL: "Personalized aortic pressure waveform estimation from brachial pressure waveform using an adaptive transfer function", COMPUTERS IN BIOLOGY AND MEDICINE, vol. 155, 1 March 2023 (2023-03-01), page 106654, XP093173568, US ISSN: 0010-4825, DOI: 10.1016/j.compbiomed.2023.106654 * abstract; figure 1 * | 1-15 | |
| A | CN 103 479 343 B (UNIV SHANGHAI JIAOTONG) 25 February 2015 (2015-02-25) * paragraphs [0034] - [0043] * | 6 | TECHNICAL FIELDS SEARCHED (IPC) A61B |
| A | MARTIN C BARUCH ET AL: "Validation of the pulse decomposition analysis algorithm using central arterial blood pressure", BIOMEDICAL ENGINEERING ONLINE, BIOMED CENTRAL LTD, LONDON, GB, vol. 13, no. 1, 8 July 2014 (2014-07-08), page 96, XP021194726, ISSN: 1475-925X, DOI: 10.1186/1475-925X-13-96 * abstract * | 6 | |

-/--

The present search report has been drawn up for all claims

| Place of search | Date of completion of the search | Examiner |
|---|---|---|
| The Hague | 17 June 2024 | Chacon Caldera, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

## EUROPEAN SEARCH REPORT

**Application Number**

EP 24 17 1963

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SCHMID PETER J: "Dynamic Mode Decomposition and Its Variants", ANNUAL REVIEW OF FLUID MECHANICS., vol. 54, no. 1, 5 January 2022 (2022-01-05), pages 225-254, XP093174998, US ISSN: 0066-4189, DOI: 10.1146/annurev-fluid-030121-015835 * Section 4.4. Other Application Areas * ----- | 7 | |
| A | US 2020/337648 A1 (SARIPALLI VENKATA RATNA [US] ET AL) 29 October 2020 (2020-10-29) * paragraph [0125] * ----- | 7 | |

|  |  |
|---|---|
|  | TECHNICAL FIELDS SEARCHED (IPC) |

The present search report has been drawn up for all claims

| Place of search | Date of completion of the search | Examiner |
|---|---|---|
| The Hague | 17 June 2024 | Chacon Caldera, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 4 640 144 A1**

## ANNEX TO THE EUROPEAN SEARCH REPORT
## ON EUROPEAN PATENT APPLICATION NO.

EP 24 17 1963

17-06-2024

| Patent document cited in search report | Publication date | Patent family member(s) | Publication date |
|---|---|---|---|
| US 2019298191 A1 | 03-10-2019 | US 2019298191 A1 | 03-10-2019 |
| | | WO 2017184700 A1 | 26-10-2017 |
| CN 103479343 B | 25-02-2015 | NONE | |
| US 2020337648 A1 | 29-10-2020 | CN 111863236 A | 30-10-2020 |
| | | KR 20200124610 A | 03-11-2020 |
| | | US 2020337648 A1 | 29-10-2020 |
| | | US 2020342362 A1 | 29-10-2020 |
| | | US 2020342968 A1 | 29-10-2020 |

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Relationships between blood pressure and systolic time-intervals: a lumped-model simulation study. **AUBERT, X. L.** ; **MUEHLSTEFF, J.** ; **ZHU, C**. In 2007 29th Annual International Conference of the IEEE Engineering in Medicine and Biology Society. IEEE., August 2007, 1707-1710

- **J. ALASTRUEY** ; **K. H. PARKER** ; **S. J. SHERWIN**. Arterial pulse wave haemodynamics. *BHR Group - 11th Int. Conf. Press. Surges*, 2012, 401-442

- **J. P. MYNARD** ; **J. J. SMOLICH**. One-Dimensional Haemodynamic Modeling and Wave Dynamics in the Entire Adult Circulation. *Ann. Biomed. Eng.*, June 2015, vol. 43 (6), 1443-1460

- **P. REYMOND** ; **F. MERENDA** ; **F. PERREN** ; **D. RÜFENACHT** ; **N. STERGIOPULOS**. Validation of a one-dimensional model of the systemic arterial tree. *Am. J. Physiol.-Heart Circ. Physiol.*, 2009, vol. 297 (1), H208-H222

- **J. ALASTRUEY** ; **N. XIAO** ; **H. FOK** ; **T. SCHAEFF-TER** ; **C. A. FIGUEROA**. On the impact of modelling assumptions in multi-scale, subject-specific models of aortic haemodynamics. *J. R. Soc. Interface*, June 2016, vol. 13 (119), 20160073

- **M. WILLEMET** ; **P. CHOWIENCZYK** ; **J. ALAS-TRUEY**. A database of virtual healthy subjects to assess the accuracy of foot-to-foot pulse wave velocities for estimation of aortic stiffness. *Am. J. Physiol.-Heart Circ. Physiol.*, 2015, vol. 309 (4), H663-H675

- **P. H. CHARLTON** ; **J. MARISCAL HARANA** ; **S. VENNIN** ; **Y. LI** ; **P. CHOWIENCZYK** ; **J. ALAS-TRUEY**. *Pulse Wave Database (PWDB): A database of arterial pulse waves representative of healthy adults*, 2019